(19) Europäisches Patentamt
European Patent Office
Office européen des brevets

(11) EP 0 404 546 B1

(12) EUROPEAN PATENT SPECIFICATION

(45) Date of publication and mention
of the grant of the patent:
22.10.1997 Bulletin 1997/43

(51) Int Cl.6: C12N 15/16, C07K 14/59

(21) Application number: 90306743.7

(22) Date of filing: 20.06.1990

(54) **Heteropolymeric protein production**

Herstellung von heteropolymeren Proteinen

Production de protéines hétéropolymères

(84) Designated Contracting States:
AT BE CH DE DK ES FR GB GR IT LI LU NL SE

(30) Priority: 20.06.1989 US 368628

(43) Date of publication of application:
27.12.1990 Bulletin 1990/52

(60) Divisional application: 96102098.9

(73) Proprietor: GENZYME CORPORATION
Framingham, Massachusetts 01701 (US)

(72) Inventors:
• Kelton, Christie A.
Hopkinton, Massachusetts 01748 (US)
• Nugent, Noreen P.
Framingham, Massachusetts 01701 (US)
• Cheppel, Scott C.
Jamaica Plain Massachusetts 02130 (US)

(74) Representative: Sheard, Andrew Gregory et al
Kilburn & Strode
30, John Street
London WC1N 2DD (GB)

(56) References cited:
EP-A- 0 227 064          EP-A- 0 260 148
WO-A-90/08528

• Mol.Endocrinology vol.2, pages 32-39
(Wondisford et al, 1988)
• Biochem.Biophys.Res.Comm., vol.149, pages
1149-1155 (Watanabe et al, 1987)
• Gene vol.73, pages 489-497 (Tatsumi et al., 1988)
• The Journal of Cell Biology, vol.106, no.4, april
1988, pages 1049-1059; M.Matzuk and I.Boime:
"The role of the asparagine-linked
oligosaccharides of the alpha subunit in the
secretion and assembly of human chorionic
gonadotropin"
• Proceedings of the National Academy of
Sciences of USA, vol.82, november 1985,
Washington US, pages 7280-7283; D.Kaetzel et
al: "Expression of biologically active bovine
lueinizing hormone in Chinese hamster ovary
cells"
• Febs Letters, vol.188, no.2, september 1985,
pages 394-400; Y.Hayashizaki et al: "Molecular
cloning of the human thyrotropin-beta subunit
gene
• Gene, vol.73, 1988, Amsterdam NL, pages
295-304; W.Chan et al: "Stability of group I intron
RNA in escherichia coli and its potential"

Remarks:
Divisional application 96102098.9 filed on 12/02/96.

EP 0 404 546 B1

## Description

This invention relates to expression systems for the production of heteropolymeric proteins from transformed mammalian cells and more particularly concerns novel expression systems and vectors for the production of dimeric glycoprotein hormones.

Transfection of the α and β subunit cDNA clones into cultured mammalian cells has characteristically resulted in low gonadotropin expression levels. This has seriously impeded the production of these hormones on a commercial scale.

It is one aspect of the present invention to provide commercially practical methods for the production of such hormones.

While some genes, such as β-globin (1, 2) and immunoglobulin genes (3-5), require introns for optimal mRNA production, other genes, such as thymidine kinase (6), do not. Intron-dependent increases in gene expression can result from either non-transcriptional (e.g. globin genes) or transcriptional (e.g. immunoglobulin genes) mechanisms.

Isolation of the genes which encode the human and bovine common α, FSHβ, LHβ, and human TSHβ subunits has been reported (8-14). Ramabhadran et al. (15) first described transfection with and subsequent expression of the human alpha subunit cDNA in mouse cells. Several groups have since reported successful expression of dimeric glycoprotein hormones by transfection of cultured mammalian cells. Some of these groups (16, 17) employed cDNA clones while others (14, 18, 19) have used intron-containing cDNA or genomic sequences.

USSN 548,228 and USSN 696,647 describe the use of cDNA clones (without introns) to produce gonadotropins in cultured mammalian cells. While those expression systems yield biologically active molecules, the yield of the transformed mammalian cells is generally lower than described.

It is another aspect of the present invention to provide improved expression systems, useful with, for example, gonadotropins, which result in higher yield.

Matzuk and Boime (18a) mention that an intron inserted into the coding region of the human α subunit cDNA improved expression results compared with the use of cDNA clones but provided no data to support that contention or specific description of their methods. In a recent publication, Kaetzel and Nilson (7) reported relatively high levels of bovine LH expression in CHO cells. Their system employed genomic sequences for expression of both the α and LHβ subunits. However, the effect of genomic sequences versus cDNA sequences upon LH expression was not addressed in their paper.

It is yet another aspect of the present invention to obviate the confusion represented by the present state of the art and to provide the critical teaching necessary to derive improved vectors encoding dimeric glycoproteins and production methods utilizing such vectors.

Introns have been linked to increased mRNA accumulation in tissue culture cells for rabbit β-globin (1,2,20), E. coli gpt (20), and mouse DHFR (20, 21). Examples of genes containing introns with enhancer elements which increase transcription are the immunoglobulin genes (3-5), the rat cytochrome c gene (22), and the human pro-α1(I) collagen gene (23). Introns have also been shown to result in increased transcriptional efficiency in transgenic mice for the following genes: rat growth hormone, mouse metallothionein-I, and human β-globin (24). However, introns have no effect on the expression of these last three genes when they are transfected into cultured mammalian cells.

It has been shown that expression levels can be influenced by different 3' untranslated and polyadenylation regions (24, 25). For example, higher expression levels of a galK marker gene result if the bovine growth hormone polyadenylation region is used for transcription termination rather than the SV40 early or human collagen polyadenylation regions (24).

The present inventors have discovered that expression systems employing the use of α subunit genomic sequences, or α subunit cDNA constructions with an added intron, which significantly and surprisingly enhance dimeric glycoprotein hormone production in mammalian cells. This discovery will facilitate the development of processes for high-yield production of dimeric glycoprotein hormones which share the common α subunit. These include chorionic gonadotropin (CG), follicle stimulating hormone (FSH), luteinizing hormone (LH), and thyroid stimulating hormone (TSH).

It has also been unexpectedly discovered that TSHβ subunit gene expression is intron-dependent. Characterization of the genomic regions necessary for optimal expression of the TSHβ subunit now made possible by the instant invention provide critical and specific information regarding the development of a process for efficient production of dimeric TSH.

According to the present invention, there is provided a method for the production of thyroid stimulating hormone (TSH), the method comprising the steps of:

a) providing a vector comprising a promoter and a structural gene encoding at least a portion of the β subunit of TSH and comprising a terminating sequence, said vector optionally also comprising a second structural gene encoding at least a portion of the a subunit of TSH; wherein the first structural gene comprises at least one intron and the second structural gene (if present in said vector) also comprises at least one intron;

b) if the vector does not include said second structural gene, providing a second vector comprising said second structural gene with at least one intron;

c) transforming host cells with the vector(s) to thereby form stably transfected cells; and

d) culturing the transformed cells under conditions whereby TSH is produced.

Various aspects of this invention are set forth in claims 2 to 12.

Preferred embodiments and examples of the present invention will now be described with reference to the accompanying drawings. Where subject matter described does not fall within the scope of the claims it is provided for information purposes only.

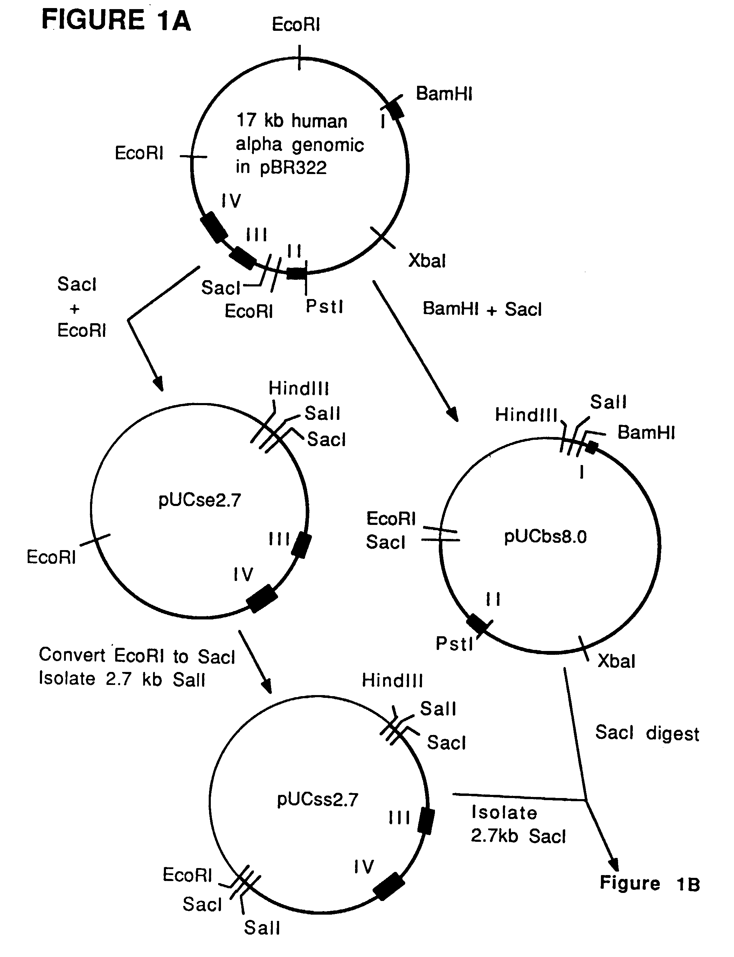

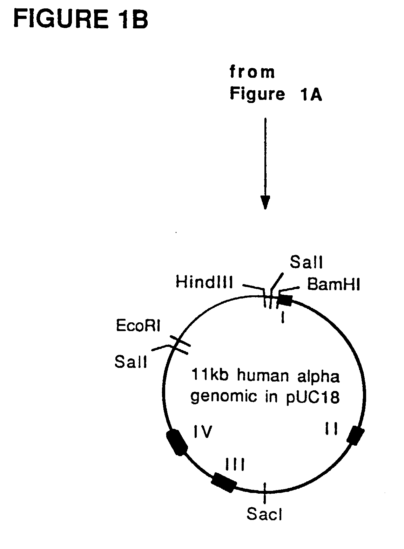

Figure 1A+B: shows the strategy used to engineer the human α gene for expression in tissue culture cells. Important restriction endonuclease sites are indicated. Filled-in boxes represent α gene exons; heavy solid lines, α gene introns; thin solid lines, pBR322 or pUC18 vector; and zig-zag lines, pUC18 polylinker regions.

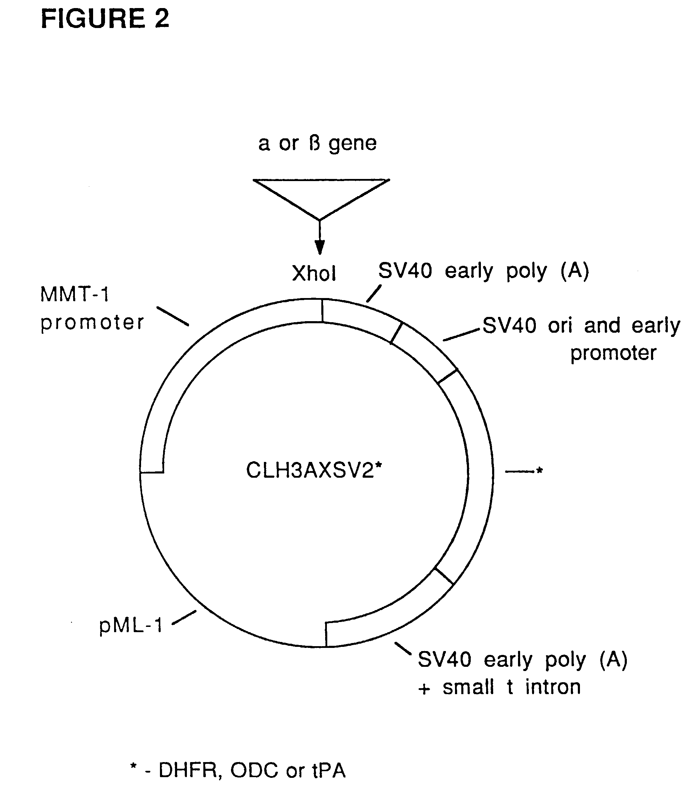

Figure 2: shows the basic expression vector, CLH3AXSV2[DHFR, ODC, or TPA], used for stable or transient transfections. The position of the XhoI site used for insertion of α or β subunit sequences is shown. Double solid lines represent sequences needed for mammalian cell expression. The relative positions of the promoter, polyadenylation, and marker gene regions are indicated. The single solid line represents the pBR322-derived pML-1 region necessary for propagation and selection (by ampicillin resistance) in E. coli.

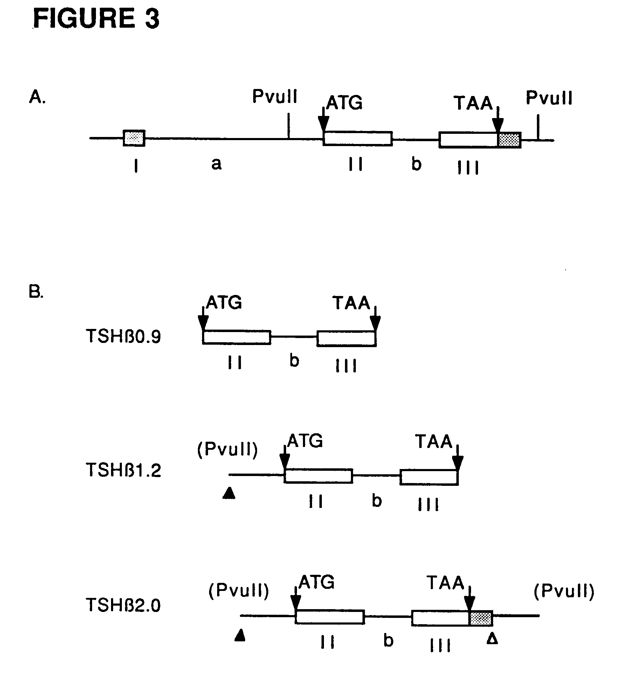

Figure 3: part A shows the full-length TSBβ gene. The positions of the three exons (I, II, and III), two introns (a, b), start ATG, and stop codon (TAA) are indicated. The PvuII sites used to isolate the fragment from which the partial constructions were derived are also indicated. Hatched boxes denote noncoding exon sequences. Part B shows the partial genomic constructions used to compare TSHβ mRNA accumulation in transiently transfected COS-7 cells. TSHβ 0.9 consists of the two coding exons separated by the endogenous IVS with all sequences upstream of the start ATG and downstream of the TAA removed (sequence shown in Table 9). TSHβ 1.2 and TSHβ 2.0 contain, in addition, about 300 base pairs of intron a and were constructed by adding a synthetic splice donor (▲) to allow splicing of the truncated intron. TSHβ 2.0 retained the endogenous polyadenylation signal (Δ) and about 0.8 kb of additional 3' flanking sequence.

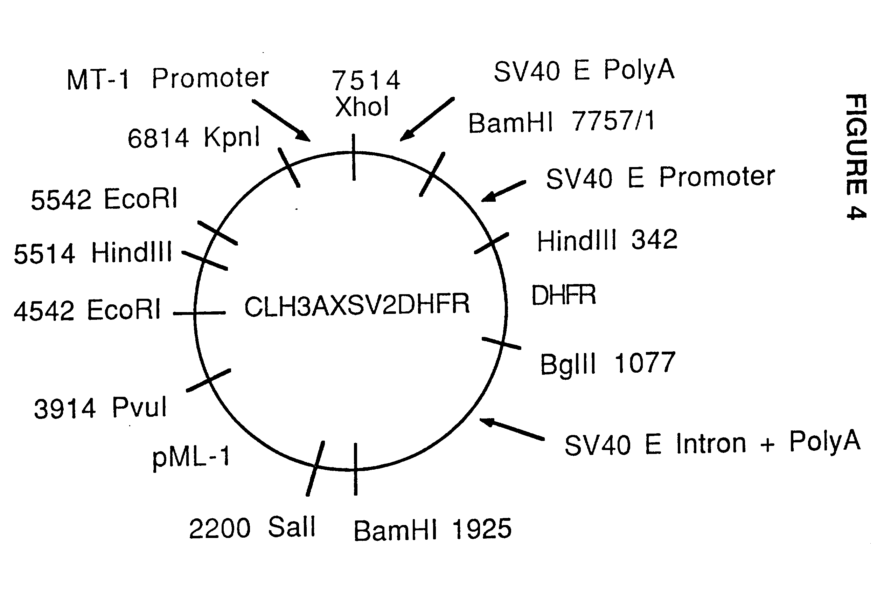

Figure 4: describes CLH3AXSV2DHFR. This vector was constructed from the following components: (i) the dehydrofolate reductase (DHFR) transcriptional unit (nucleotide numbers 1 to 1925 of Figure 4) which consists of the SV40 early region promoter (33, 34), the mouse DHFR gene (REFS) and the SV40 small T intron and early region polyadenylation signal sequences (33, 34); (ii) the bacterial plasmid vector sequences of pML (nucleotide numbers 2201 to 4542 of Figure 4) derived from the pBR322 vector (29) from which a 1370 base pair sequence has been deleted (32); and (iii) the metallothionein promoter (nucleotide numbers 4542 to 7514 of Figure 4) derived from the mouse metallothionein-1 gene (30, 31) from which the introns and polyadenylation signal sequences have been removed; and (iv) the SV40 early region polyadenylation signal sequences (nucleotide numbers 7514 to 7751 of Figure 4) (33, 34). The tPA analogues were inserted into the vector as a SalI fragment at the unique XhoI site. The orientation of the insert relative to the promoter and polyadenylation sequences was determined by restriction enzyme analysis.

Example 1: Plasmid constructions for the expression of the common α and FSHβ subunits.

A. The complete human α subunit genomic clone can be conveniently obtained from a number of sources as a 17 kilobase pair (kb) EcoRI insert in pBR322. Because the α promoter has been shown to be tissue specific and would be unlikely to function efficiently in the tissue culture cells commonly used for heterologous gene expression, steps were taken to remove all 5' flanking sequences. The presence of internal EcoRI sites necessitated several subcloning steps prior to assembly of the trimmed genomic sequence. The engineering strategy advantageously used is diagrammed in Figure 1. Two pUC18 subclones were constructed, the first with the 8.0 kb BamHI-SacI 5' piece (pUCbs8.0), and the second with the 3' 2.7 kb SacI-EcoRI piece (pUCse2.7). To generate a terminus compatible with the XhoI cloning sites in our expression vectors, pUCse2.7 was digested with EcoRI, the ends were blunted by treatment with the Klenow fragment of E. coli DNA polymerase I, and then SalI linkers were attached in a ligation reaction. Subsequent SalI restriction endonuclease digestion of the reaction mixture yielded a 2.7 kb human α SalI piece in addition to the 2.6 kb vector fragment. The 2.7 kb human α SalI piece was gel purified and re-inserted into into the SalI site of pUC18 (pUCss2.7). A clone was chosen which contained the SalI insert in the orientation which permitted isolation of the 2.7 kb α fragment as a SacI piece. This was gel purified and then inserted into the SacI site of pUCbs8.0 to assemble the complete coding sequence of the human α gene as an 11 kb insert in pUC18. The insert could be excised as a SalI fragment by virtue of a pre-existing SalI site in the pUC18 polylinker at the 5' end of the gene and the converted SalI site (from EcoRI) at the 3' end of the gene. The completed genomic α expression construction, henceforth referred to as the "full-length genomic α" sequence, included part of exon I (less the first 35 nucleotides which comprise the 5' untranslated region of the mRNA), all of exons II, III, and IV as well as the intervening sequences, and approximately two kilobase pairs (kb) of 3' flanking sequence. This was inserted into the XhoI site of the CLH3AXSV2DHFR expression vector (Figure 2) so that transcription was directed by the mouse metallothionein-I (MT-I) promoter. The expression vector also contained the mouse dihydrofolate reductase (DHFR) gene for a selectable and amplifiable marker.

B. The human $\alpha$ subunit cDNA was engineered for expression by digesting the full-length clone with NcoI, which spans the start ATG, and HindIII, which cleaves in the 3' untranslated region 215 base pairs (bp) downstream of the TAA stop codon. A 5' SalI site and Kozak consensus sequence (27) was provided by synthetic oligonucleotides, and a 3' SalI site by attaching linkers as described above. The DNA sequence of the engineered $\alpha$ subunit cDNA clone, which is approximately 600 bp in length, is shown in Table 7. This was inserted into the XhoI site of the CLH3AXSV2DHFR expression vector (Figure 2). The endogenous 5' untranslated region and 3' polyadenylation signal were removed from the cDNA clone in the process of engineering and therefore were supplied by vector sequences: the MT-I promoter and the simian virus 40 (SV40) early polyadenylation signal, respectively.

C. The human FSHβ partial genomic clone used in this study was a 2.0 kb DdeI-Sau3A segment which contained the protein coding region of exon II in addition to 40 bp of sequence upstream of the start ATG, the protein coding region of exon III, and the 1.6 kb intron which separates the two exons (Table 7). The 5' DdeI and the 3' Sau3A sites had formerly been converted to EcoRI and BamHI sites, respectively, and therefore were not compatible with the current expression vectors. The partial FSHβ genomic clone was therefore supplied with SalI termini by blunting as described above and attaching commercially prepared SalI linkers. The SalI piece was then inserted into the XhoI site of CLH3AXSV20DC (Figure 2), an expression vector structurally similar to CLH3AXSV2DHFR except that the DHFR coding region was replaced with that for murine ornithine decarboxylase (ODC).

Example 2: Comparison of the full-length genomic $\alpha$ and cDNA $\alpha$ CLH3AXSV2DHFR transcription units in stable transfections.

The genomic $\alpha$ and cDNA $\alpha$ CLH3AXSV2DHFR constructions were compared by cotransfection of each $\alpha$ expression plasmid with the human FSHβ CLH3AXSV20DC expression plasmid and measurement of FSH dimer production.

A. Twenty-four hours before the transfections were to be done, 100 millimeter dishes were seeded with 7 X $10^5$ DUKX CHO cells (DHFR-minus). Calcium phosphate precipitations were obtained by adding 31 microliters ($\mu$l) of 2 molar (M) CaCl$_2$ to the plasmid DNA suspended in 500 $\mu$l of transfection buffer [14 millimolar (mM) NaCl, 5 mM KCl, 0.7 mM Na$_2$HPO$_4$, 5.5 mM dextrose, and 20 mM HEPES (pH 7.5)]. Plasmid DNA amounts used were $\alpha$ (cDNA or genomic): 10 $\mu$g, and FSHβ: 30 $\mu$g. The precipitates were allowed to form for 45 minutes at room temperature. The culture medium was removed from the cells and replaced with the DNA precipitate. After allowing the cells to sit at room temperature for 20 minutes, 5 ml of culture medium was added to each dish and incubation was continued at 37°C for 4 hours. The cells were then shocked for 3.5 minutes in 15% glycerol at 37°C. After incubation for 48 hours at 37°C in culture medium the cells were split 1:10 and selection medium containing 0.02 methotrexate (MTX) was added. The selection medium used was $\alpha$-minus modified Eagle's medium supplemented with 10% dialyzed fetal bovine serum and 1% L-glutamine. Ten to fourteen days later, foci were visible and were transferred to 24-well plates. Culture media from these were assayed for FSH dimer expression by using a specific monoclonal-based radioimmunoassay (Serono Diagnostics, Randolph, MA). Positive clones were transferred to T-25 flasks in selection medium which contained an increased MTX concentration of 0.1 $\mu$M. When the cultures reached confluence the media were again assayed for FSH dimer and the cells were counted to calculate the picogram per cell per 24 hour expression levels.

B. Human FSH dimer secretion levels measured in seven randomly selected clones from each of the human $\alpha$ genomic/FSHβ and human $\alpha$ cDNA/FSHβ cotransfections are presented in Table 1.

TABLE 1

| $\alpha$ GENOMIC-DHFR/FSHβ-ODC | | $\alpha$ cDNA-DHFR/FSHβ-ODC | |
|---|---|---|---|
| GFSH Cell Line | pg/cell/24h in 0.1 $\mu$M MTX | CFSH Cell Line | pg/cell/24h in 0.1 $\mu$M MTX |
| 1 | 0.43 | 18 | 0.003 |
| 3 | 1.95 | 37 | 0.03 |
| 4 | 0.50 | 51 | 0.08 |
| 5 | 0.70 | 57 | 0.02 |
| 7 | 1.44 | 60 | 0.056 |
| 8 | 1.18 | 66 | 0.051 |

TABLE 1 (continued)

| α GENOMIC-DHFR/FSHβ-ODC | | α cDNA-DHFR/FSHβ-ODC | |
|---|---|---|---|
| GFSH Cell Line | pg/cell/24h in 0.1 μM MTX | CFSH Cell Line | pg/cell/24h in 0.1 μM MTX |
| 9 | 2.56 | 70 | 0.013 |
| Avg. | $\overline{1.25}$ | Avg. | $\overline{0.04}$ |

The results show that FSH dimer expression is greatly enhanced in cells transfected with the full-length genomic α subunit sequence. The averaged expression levels indicate that the surprisingly large magnitude of the enhancement seen in this particular experiment was approximately thirty-fold.

C. To further demonstrate the superiority of the full-genomic α sequence, stable cell lines were transfected with the CLH3AXSV2DHFR expression vector that contained either the human α cDNA or the human α genomic clone. Expression rates of the free α subunit were compared. In all cases, the expression of human α subunit, as determined by a sensitive and specific radioimmunoassay, was never greater than 0.05 pg/cell/24h for the cDNA-containing cell lines. As detailed in Table 2, cells that were transfected with the genomic α clone expressed 5-20 times greater levels of the protein:

## TABLE 2

| Alpha Cell Line | Expression Level pg/cell/24h |
|---|---|
| 2 | 0.32 |
| 10 | 0.36 |
| 12 | 0.57 |
| 17 | 0.37 |
| 18 | 0.39 |
| 38 | 1.28 |
| 47 | 0.63 |
| 51 | 0.26 |

Example 3: Thyroid Stimulating Hormone Constructions

To demonstrate the effectiveness and broad application of this invention, stably transfected CHO cell lines were prepared by cotransfection of the full-length human genomic α sequence in CLH3AXSV2DHFR with a partial genomic TSHβ sequence in CLH3AXSV20DC. The partial genomic TSHβ fragment used in this experiment consisted of the protein coding regions of exon II and III, and the 0.5 kb intron which separated the two exons (Table 9). All 5' and 3' regions flanking the protein coding sequence of TSHβ were removed in this particular construction. Following cotransfection with the two expression vectors, stable cell lines were cultured and analyzed for their ability to express TSH. Expression levels of the dimer, as determined by a sensitive and specific radioimmunoassay, are listed in Table 3. Previous studies with the transfection of the cDNA for the α subunit with the genomic TSHβ clone had demonstrated that expression levels were usually below the sensitivity of the radioimmunoassay, usually less than 0.02 pg/cell/24h.

TABLE 3

| TSH Cell Line | Expression Rate pg/cell/24h |
|---|---|
| 7 | 0.19 |
| 8 | 0.22 |
| 10 | 0.24 |

TABLE 3 (continued)

| TSH Cell Line | Expression Rate pg/cell/24h |
|---|---|
| 12 | 0.24 |
| 37 | 0.13 |
| 48 | 2.97 |

TSH production, then, like FSH production, can be greatly enhanced by use of the full genomic α sequence, rather than the α cDNA sequence, in mammalian cell transfections. In this experiment the range of TSH production enhancement was 6 to 100 fold.

Example 4: Introns and Expression Enhancement

The human α genomic construction differed from the human α cDNA construction not only in that it contained introns, but also in that it contained endongenous 5' untranslated sequence, the endogenous polyadenylation signal, and additional 3' flanking sequences. Therefore, one could not infer from the results of the previous set experiments which genomic regions contributed to the enhanced expression.

A. To determine if the introns within the genomic α sequence were responsible for the elevated α subunit levels, we inserted a 2 kb XbaI-PstI portion of the human α intron A between the mouse MT-I 5' untranslated region and the α cDNA sequence in the CLH3AXSV2TPA vector (Figure 2). The truncated intron retained the endogenous splice acceptor, but the splice donor was supplied by a synthetic oligonucleotide.

B. Another plasmid was constructed to test the effect of a heterologous intron on α subunit expression. In this construction, a 130 bp intron from the MOPC41 immunoglobulin κ gene (5) was inserted between the mouse MT-I 5' untranslated region and the α cDNA sequence. No transcriptional enhancer elements were included in this particular intervening sequence.

C. The intron-containing α cDNA constructs were compared to the original α cDNA construct and the full length genomic α sequence by transient transfection of COS-7 cells with the plasmid DNA and analysis of mRNA levels by northern blotting. In this experiment, the tissue plasminogen activator (tPA) cDNA served as an internal standard and was used to correct for the variations in the transfection efficiency of different plasmid constructions and thus normalize measured α subunit mRNA levels. Transfections were in duplicate using the DEAE-dextran protocol of Seed and Aruffo (28). Two days after transfection, total cellular RNA was isolated from the cells. The RNA (5 micrograms) was fractionated on formaldehyde gels and then transferred to nylon membranes using standard northern blotting techniques. The membranes were then hybridized to either a $^{32}$P-labeled human or α a $^{32}$P-labeled tPA probe and the resulting signals were quantitated on a Betascope Model 603 blot analyzer (Betagen Corp., Waltham, MA). Normalized α mRNA values for relative comparisons were calculated by dividing the number of α counts by the number of tPA counts and then averaging the numbers obtained for duplicate samples. Results of the experiment are shown in Table 4.

TABLE 4

| Plasmid Construction | | Betascope counts | | Normalized Values (α/tPA) | Average of Duplicates |
|---|---|---|---|---|---|
| | | α probe | tPA probe | | |
| 1. α genomic | A | 6315 | 958 | 6.6 | 7 |
| | B | 7836 | 1177 | 6.7 | |
| 2. α cDNA + α IVS | A | 25353 | 1294 | 19.6 | 21 |
| | B | 34616 | 1559 | 22.2 | |
| 3. α cDNA + Ig IVS | A | 31743 | 1690 | 18.8 | 17 |
| | B | 37495 | 2327 | 16.1 | |
| 4. α cDNA | A | 3891 | 2608 | 1.5 | 1 |

TABLE 4 (continued)

| Plasmid Construction | | Betascope counts | | Normalized Values (α/tPA) | Average of Duplicates |
|---|---|---|---|---|---|
| | | α probe | tPA probe | | |
| | B | 3671 | 3341 | 1.1 | |

The results indicated that the cDNA constructions which contained either the human α intron (No.2) or the immunoglobulin intron (No. 3) were more efficient as the full length genomic sequence (No. 1) in the accumulation of α subunit mRNA. Specifically, the normalized α subunit mRNA levels were 7- to 21-fold higher than those produced by the cDNA construct (No. 4) without an intron. We have therefore unexpectedly discovered that introns, and not the 5' or 3' regions which flank the α subunit protein coding sequence, are primarily responsible for the increased expression levels and that the effect is due, at least in part, to an increased accumulation of α subunit mRNA.

Example 5: Expression Enhancement Not Due to Increased Transcription Rates.

Nuclear runoff transcription assays were used to determine if the high level of genomic α-induced mRNA accumulation was due to an increased transcription rate. Transient transfection of COS-7 cells with the α genomic and α cDNA-containing CLH3AXSV2TPA plasmids was done as described in Example 4. Standard methods were used in the preparation of nuclei and for the runoff transcription assay (Current Protocols in Molecular Biology; Ausubel, F. M. et al., ed.; John Wiley & Sons, NY, NY). The DNA probes for α, DHFR, and tPA were gel purified insert sequences and approximately 0.25 μg of each were slot blotted in duplicate onto nitrocellulose membranes. The membranes were hybridized to [$^{32}$P-UTP] labeled nuclear runoff RNA prepared from COS-7 cells that had been transfected with no DNA (mock), the full-length human α genomic clone in CLH3AXSV2TPA, or the human α cDNA clone in CLH3AXSV2TPA. The hybridization signal was quantitated on a Betascope Model 603 blot analyzer (Betagen, Corp., Waltham, MA). Normalized values for the transcription rate were obtained by dividing the averaged α counts by the averaged tPA counts. The monkey DHFR nuclear runoff RNA should not hybridize to the mouse DHFR DNA probe sequence in the conditions used for this experiment, and therefore serves as a negative control. The relative transcription rate results are summarized in Table 5, below.

TABLE 5

| | Nuclear runoff RNA | | | | | |
|---|---|---|---|---|---|---|
| | Mock COS-7 | | α cDNASV2TPA | | αgenomic/SV2TPA | |
| DNA Probes | 1 | 2 | 1 | 2 | 1 | 2 |
| α cDNA | 119 | 160 | 2093 | 2155 | 492 | 620 |
| tPA | 387 | 413 | 18022 | 17667 | 8486 | 7388 |
| DHFR | 230 | 307 | 54 | 0 | 32 | 765* |
| Rate Ratios: | | | | | | |
| α/tPA | | - | | 0.12 | | 0.07 |

*Non-specific background rendered this value artifically high

In this particular experiment the runoff transcription rate of the α cDNA (0.12) was surprisingly higher than that of the genomic α sequence (0.07). An increased transcription rate is therefore not the mechanism by which the genomic α sequence generates higher α subunit expression levels.

Example 6: TSHβ Subunit Expression is Intron-dependent.

A. The full-length genomic TSHβ gene (14a) is diagrammed in Table 3, Part A. The positions of the three exons (I, II, and III), two introns (a, b), start ATG, and stop codon (TAA) are indicated. The PvuII sites were separated by approximately 2 kb of DNA sequence which included exons II and III and contained the complete protein coding sequence for TSHβ. The TSHβ partial genomic constructions used in this study were derived from the 2 kb PvuII fragment and are diagrammed in Table 3B. TSHβ 0.9 (0.9 kb) was the same construct used in stable transfections in Example 1 and consisted of the two coding exons separated by the endogenous intervening sequence with all sequences upstream of the start ATG and downstream of the TAA removed. TSHβ 1.2 (1.2 kb) and TSHβ 2.0 (2.0 kb) contained, in addition, about 300 base pairs of intron A and were constructed by adding a synthetic splice

7

donor to allow splicing of the truncated intron. TSHβ 2.0 retained the endongenous polyadenylation signal and about 0.8 kb of additional 3' flanking sequence.

B. Duplicate cultures (A, B) of COS-7 cells were transfected, using the protocol described in Example 4, with CLH3AXSV2DHFR plasmids which contained one of the following partial genomic fragments: (1) TSHβ 0.9, (2) TSHβ 1.2, or (3) TSHβ 2.0. The genes were inserted into the Xhol cloning site so that transcription would be initiated by the MT-I promoter and, in the case of TSHβ 0.9 and TSHβ 1.2, terminated by the SV40 early polyadenylation signal. After 48 hours of incubation, total cellular RNA was isolated from the cells. The RNA (9 micrograms) was fractionated on formaldehyde gels and then transferred to nylon membranes using standard northern blotting techniques. The membranes were hybridized to either a $^{32}$P-labeled mouse MT-I probe (to detect the TSHβ mRNA which also contains about 50 base pairs MT-I 5' untranslated sequence) or a $^{32}$P-labeled DHFR probe (to compare transfection efficiencies). The resulting signals were quantitated on a Betascope Model 603 blot analyzer (Betagen Corp., Waltham, MA). Normalized TSHβ mRNA values for relative comparisons were calculated by dividing MT-I counts by DHFR counts. Normalized values obtained for duplicate transfections were then averaged and divided by the number obtained for TSHβ 0.9. The comparative results obtained for accumulation of mRNA are summarized in Table 6.

TABLE 6

| Plasmid Construction | | Betascope Counts | | Normalized Values (MT/DHFR) | Relative Values (averaged) |
|---|---|---|---|---|---|
| | | MT Probe | DHFR Probe | | |
| 1. TSHβ 0.9 | A | 983 | 3985 | 0.25 | 1 |
| | B | 834 | 4118 | 0.20 | |
| 2. TSHβ 1.2 | A | 4480 | 254 | 17.6 | 60 |
| | B | 2748 | 285 | 9.6 | |
| 3. TSHβ 2.0 | A | 1381 | 1209 | 1.2 | 5 |
| | B | 1632 | 1254 | 1.3 | |

C. The results indicated that the two constructs which retained a portion of the first intron (TSHβ 1.2 and TSHβ 2.0) yielded 5-60 fold higher mRNA accumulation than did the construct which contained no sequences from the first intron. Therefore TSHβ gene expression, like α subunit gene expression, is intron-dependent. Of interest was the unexpected observation that intron B, which was present in all the constructs and was in the protein coding region, did not confer optimal enhancement. This suggested either that there are specific sequences in the first intron of the TSHβ gene which increased mRNA accumulation or, more likely, that the position of the intron (close to the 5' end of the mRNA) was the crucial factor. That intron-dependent gene expression may be affected by the placement of the intron in the transcription unit is supported by preliminary experiments with the human α cDNA. Insertion of the κ immunoglobulin gene intron in the 3' untranslated region of the mRNA, between the α cDNA and the SV40 polyadenylation region, resulted in no increased mRNA accumulation when compared to the unaltered α cDNA.

D. Since TSHβ 1.2 yielded 10-fold higher amounts of mRNA than TSHβ 2.0, it is probable that the endogenous polyadenylation signal and 3' flanking sequence carried on TSHβ 2.0 are not required for efficient mRNA formation, and may actually be detrimental. Alternatively, the disparity in mRNA accumulation between TSHβ 1.2 and TSHβ 2.0 may be related to the different distances between the MT-I promoter and the SV40 early promoter (driving the DHFR) in the two plasmid constructions. The closer proximity (0.8 kb) of the two promoters in the TSRβ 1.2 construction may permit increased interaction between the SV40 enhancer elements and the MT-I promoter, thereby increasing the rate of transcription from the latter.

Because of the similarity of α and TSHβ gene structure among various mammals, we expect that our findings based on the human α and TSHβ subunits apply equally to those of other species, such as the bovine, equine, porcine, baboon and monkey. Likewise, LHβ and FSHβ subunit genes may show a similar dependence on genomic DNA structure for optimal expression and these requirements should apply across species.

Construction of expression vectors will be advantageously simplified by application of the knowledge that certain genomic regions, such as introns, are important for mRNA accumulation, and that the position of these regions in the transcription unit may be important. For example, these genomic regions may be subcloned or synthesized and included in constructions with the α and β subunit cDNA sequences. The cDNA clones are often easier to obtain and to engineer than the much larger genomic clones. Thus, this discovery will simplify the development of cell lines which produce high levels of hormone and, ultimately, will permit large scale production of these proteins at a lower cost. These and other aspects and variations of the present invention will now be readily apparent to those skilled in the art and are not deemed to depart from either the spirit or the scope of the present invention.

References

1. Hamer, D.H. and Leder, P. (1979) Cell 17, 737-747

2. Hamer, D.H. and Leder, P. (1979) Cell 18, 1299-1302

3. Banerji, J. et al. (1983) Cell 33, 729-740

4. Gillies, S.D. et al. (1983) Cell 33, 717-728

5. Queen, C. and Baltimore, D. (1983) Cell 33, 741-748

6. Gross, M. K. et al. (1987) Mol. Cell. Biol. 7, 4576-4581

7. Kaetzel, D.M. and Nilson, J.H. (1988) J. Biol. Chem. 263, 6344-6351

8. Fiddes, J.C. and Goodman, H.M. (1981) J. Mol. Appl. Genetics 1, 3-18

9. Goodwin, R.G. et al. (1983) Nucl. Acids Res. 11, 6873-6882

10. Virgin, J. et al. (1985) J. Biol. Chem. 260, 7072-7077

11. Talmadge, K. et al. (1984) Nature 307, 37-40

12. Kim, K.E. et al. (1988) DNA 7, 227-233

13. Watkins, P.C. et al. (1987) DNA 6, 205-212

14. Wondisford, F.E. et al. (1988) Mol. Endo. 2, 32-39

14a. Wondisford, F.E. et al. (1988) J. Biol. Chem. 263, 12538-12542

15. Ramabhadran, T.V. et al. (1984) Proc. Natl. Acad. Sci. USA 81, 6701-6705

16. Reddy, V.B. et al. (1985) Proc. Natl. Acad. Sci. USA 82, 3644-3648

17. Watanabe, S. et al. (1987) Biochem. Biophys. Res. Comm. 149, 1149-1155

18. Matzuk, M.M. et al. (1987) Proc. Natl. Acad. Sci. USA 84, 6354-6358

18a. Matzuk, M. and Boime, I. (1988) J. Cell Biol. 106, 1049-1059

19. Kaetzel, D.M. et al. (1985) Proc. Natl. Acad. Sci. USA 82, 7280-7283

20. Buchman, A.R. and Berg, P. (1988) Mol. Cell. Biol. 8, 4395-4405

21. Lee, F. et al. (1981) Nature 294, 228-232

22. Evans, M.J. and Scarpulla, R.C. (1988) Mol. Cell. Biol. 8, 35-41

23. Rossouw, C.M.S. et al. (1987) J. Biol. Chem. 262, 15151-15157

24. Brinster, R.L. et al. (1987) Proc. Natl. Acad. Sci. USA 85, 836-840

25. Pfarr, D.S. et al. (1986) DNA 5, 115-122

26. Ross, J. and Kobs, G. (1986) J. Mol. Biol. 188, 579-593

27. Kozak, M. (1984) Nucl. Acids Res. 12, 857-872

28. Seed, B. and Aruffo, A. (1987) Proc. Natl. Acad. Sci. USA 84, 3365-3365

29. Bolivar, F., Rodriguez, R.L., Green, P.J., Betlach, M.C., Heynecker, H.L., Boyer, H.W., Crosa, G.F. and Falkow, S. (1977) Gene 2:95.

30. Glanville, N., Durnham, D.M. and Palmiter, R.D. (1981) Nature 292:267-269.

31. Hamer, D.H. and Walling, M. (1982) J. Mol. Appl. Gen. 1:273-288.

32. Lusky, M. and Botchan, M. (1981) Nature 283:79-81.

33. Reddy, V.B., Thimmapaya, B., Dhar, R., Subramanian, K.N., Zain, B.S., Pan, J., Ghosh P.K., Celma, M.L. and Weissman, S.M. (1978) Science 200:494.

34. Tooze, J. (ed.), DNA Tumor Viruses; Molecular Biology of Tumor Viruses; 2nd Ed., Cold Spring Harbor Laboratory, New York 1981.

35. Chiang T.-R. and McConlogue, L. (1988), Mol. Cell. Biol. 8:764-769.

Except as otherwise stated, procedures and techniques used are generally as reported and conventional in the art such as is described by Maniatis et al, A Cloning Manual, Cold Spring Harbor (1982). All references cited above or in the specification are fully incorporated herein by reference.

## TABLE 7

```
                  Sal1              Kozak
                                        MetAspTyrTyrArgLysTyrAlaAlaIlePheLeuValThrLeu
    1   GTCGACGGTACCACCATGGATTACTACAGAAAATATGCAGCTATCTTTCTGGTCACATTG   60
                  Kpn1

        SerValPheLeuHisValLeuHisSerAlaProAspValGlnAspCysProGluCysThr
   61   TCGGTGTTTCTGCATGTTCTCCATTCCGCTCCTGATGTGCAGGATTGCCCAGAATGCACG   120

        LeuGlnGluAsnProPhePheSerGlnProGlyAlaProIleLeuGlnCysMetGlyCys
  121   CTACAGGAAAACCCATTCTTCTCCCAGCCGGGTGCCCCAATACTTCAGTGCATGGGCTGC   180

        CysPheSerArgAlaTyrProThrProLeuArgSerLysLysThrMetLeuValGlnLys
  181   TGCTTCTCTAGAGCATATCCCACTCCACTAAGGTCCAAGAAGACGATGTTGGTCCAAAAG   240

        AsnValThrSerGluSerThrCysCysValAlaLysSerTyrAsnArgValThrValMet
  241   AACGTCACCTCAGAGTCCACTTGCTGTGTAGCTAAATCATATAACAGGGTCACAGTAATG   300

        GlyGlyPheLysValGluAsnHisThrAlaCysHisCysSerThrCysTyrTyrHisLys
  301   GGGGGTTTCAAAGTGGAGAACCACACGGCGTGCCACTGCAGTACTTGTTATTATCACAAA   360

        Ser
  361   TCTTAAATGTTTTACCAAGTGCTGTCTTGATGACTGCTGATTTTCTGGAATGGAAAATTA   420

  421   AGTTGTTTAGTGTTTATGGCTTTGTGAGATAAAACTCTCCTTTTCCTTACCATACCACTT   480

  481   TGACACGCTTCAAGGATATACTGCAGCTTTACTGCCTTCCTCCTTATCCTACAGTACAAT   540

  541   CAGCAGTCTAGTTCTTTTCATTTGGAATGAATACAGCATTAAGCTGGTCGAC   592
                                                        Sal1
```

The engineered alpha subunit cDNA sequence. Restriction endonuclease sites and Kozak consensus region which were supplied by synthetic oligonucleotides are indicated. The alpha subunit amino acid sequence is also shown.

# EP 0 404 546 B1

## TABLE 8

```
Sal1
                                                        MetLysThrLeuGlnPheP
    1  TCAGTTTCTAGTGGGCTTCATTGTTTGCTTCCCAGACCAGGATGAAGACACTCCAGTTTT      60


       hePheLeuPheCysCysTrpLysAlaIleCysCysAsnSerCysGluLeuThrAsnIleT
   61  TCTTCCTTTTCTGTTGCTGGAAAGCAATCTGCTGCAATAGCTGTGAGCTGACCAACATCA     120


       hrIleAlaIleGluLysGluGluCysArgPheCysIleSerIleAsnThrThrTrpCysA
  121  CCATTGCAATAGAGAAAGAAGAATGTCGTTTCTGCATAAGCATCAACACCACTTGGTGTG     180


       laGlyTyrCysTyrThrArg                 1.6 kb IVS
  181  CTGGCTACTGCTACACCAGGGTAGGTACC...........TAGAGCAAGCAGTATTCA    1674


 1675  ATTTCTGTCTCATTTTGACTAAGCTAAATAGGAACTTCCACAATACCATAACCTAACTCT    1734


                     AspLeuValTyrLysAspProAlaArgProLysIleGlnLysT
 1735  CTTCTTAAACTCCTCAGGATCTGGTGTATAAGGACCCAGCCAGGCCCAAAATCCAGAAAA    1794


       hrCysThrPheLysGluLeuValTyrGluThrValArgValProGlyCysAlaHisHisA
 1795  CATGTACCTTCAAGGAACTGGTATATGAAACAGTGAGAGTGCCCGGCTGTGCTCACCATG    1854


       laAspSerLeuTyrThrTyrProValAlaThrGlnCysHisCysGlyLysCysAspSerA
 1855  CAGATTCCTTGTATACATACCCAGTGGCCACCCAGTGTCACTGTGGCAAGTGTGACAGCG    1914


       spSerThrAspCysThrValArgGlyLeuGlyProSerTyrCysSerPheGlyGluMetL
 1915  ACAGCACTGATTGTACTGTGCGAGGCCTGGGGCCCAGCTACTGCTCCTTTGGTGAAATGA    1974


       ysGlu
 1975  AAGAATAAAGATCcggatcggtcgac    2000

                              Sal1
```

The engineered human FSH beta subunit partial genomic sequence. Positions of the terminal SalI sites are indicated. Nucleotides in lower case at the 3' terminus results from attachment of synthetic linkers. The FSH beta amino acid sequence marks the coding regions of exons II and III. The dotted line represents uncharacterized intervening sequence (IVS).

12

TABLE 9

```
         Sal1          Kozak
                              MetThrAlaLeuPheLeuMetSerMetLeuPheGlyLeuAlaCys
      1  GTCGACGCCGCCACCATGACTGCTCTCTTTCTGATGTCCATGCTTTTTGGCCTTGCATGT    60

         GlyGlnAlaMetSerPheCysIleProThrGluTyrThrMetHisIleGluArgArgGlu
     61  GGGCAAGCGATGTCTTTTTGTATTCCAACTGAGTATACAATGCACATCGAAAGGAGAGAG   120

         CysAlaTyrCysLeuThrIleAsnThrThrIleCysAlaGlyTyrCysMetThrArg
    121  TGTGCTTATTGCCTAACCATCAACACCACCATCTGTGCTGGATATTGTATGACACGGGTA   180

    181  TGTAGTTCATGTCACTTCTTTTGGCTGTAAATTATATAAGCCCTGAAGAAGTCCATTCCT   240

    241  ATATAGAAAGGAAATGAAATAAATCACAACCTCATTTCCCAAATCTAATGGTTATTGGCT   300

    301  CCTTAGAAGCAGAGTACACAGGTTACAATATTATGTGAATCTACTCAGCACAATGGATAC   360

    361  GCATAATTTTATAACAGTTTTGTGTCCCAGCTTTACTTAAACCTTATCTTGTTCCCATGA   420

    421  TCAACGATGAAAGAGAGGAGGGTCTCACTTTTGTCTCTGTAGAATTCAACGTGGTTAAGT   480

    481  TGGTATTGGAGAATGGGGCTAAGCAATTCTTTCCCAGTTGTATTTGTGATGAAGGAATAT   540

    541  AAGTGAATTTATTTTTATGTTTCTATTATCTATATGTTTCCTAAAGTCCTGTCACATTAT   600

                              AspIleAsnGlyLysLeuPheLeuProLysTyrAl
    601  GCTCTCTTTTCTGTTCTTTCCCCAGGATATCAATGGCAAACTGTTTCTTCCCAAATATGC   660

         aLeuSerGlnAspValCysThrTyrArgAspPheIleTyrArgThrValGluIleProGl
    661  TCTGTCCCAGGATGTTTGCACATATAGAGACTTCATCTACAGGACTGTAGAAATACCAGG   720

         yCysProLeuHisValAlaProTyrPheSerTyrProValAlaLeuSerCysLysCysGl
    721  ATGCCCACTCCATGTTGCTCCCTATTTTTCCTATCCTGTTGCTTTAAGCTGTAAGTGTGG   780

         yLysCysAsnThrAspTyrSerAspCysIleHisGluAlaIleLysThrAsnTyrCysTh
    781  CAAGTGCAATACTGACTATAGTGACTGCATACATGAAGCCATCAAGACAAACTACTGTAC   840

         rLysProGlnLysSerTyrLeuValGlyPheSerValEnd
    841  CAAACCTCAGAAGTCTTATCTGGTAGGATTTTCTGTCTAAGTCGAC    886
                                                 Sal1
```

The engineered TSH beta subunit partial genomic sequence. Sal1 sites and Kozak consensus sequence supplied by synthetic oligonucleotides are indicated. The TSH beta amino acid sequence marks the coding regions of exons II and III.

**Claims**

1.  A method for the production of thyroid stimulating hormone (TSH), the method comprising the steps of:

    a) providing a vector comprising a promoter and a structural gene encoding at least a portion of the $\beta$ subunit of TSH and comprising a terminating sequence, said vector optionally also comprising a second structural gene encoding at least a portion of the $\alpha$ subunit of TSH; wherein the first structural gene comprises at least one intron and the second structural gene (if present in said vector) also comprises at least one intron;

    b) if the vector does not include said second structural gene, providing a second vector comprising said second structural gene with at least one intron;

c) transforming host cells with the vector(s) to thereby form stably transfected cells; and

d) culturing the transformed cells under conditions whereby TSH is produced.

2. A method as claimed in claim 1 wherein the second structural gene comprises a cDNA sequence encoding the TSH a subunit and having a 3' end and a 5' end, said second structural gene comprising an intron which is located nearer said 5' end than said 3' end of the cDNA sequence.

3. A method as claimed in claim 2 wherein the $\alpha$ subunit comprises the sequence set forth in Table 7.

4. A method as claimed in claim 2 or claim 3 wherein said cDNA encoding said $\alpha$ subunit comprises a plurality of introns, 3' and 5' flanking regions, endogenous 5' untranslated sequence and polyadenylation signal.

5. A method as claimed in claim 1 wherein the thyroid stimulating hormone is human thyroid stimulating hormone and the first and second structural genes comprise genomic DNA encoding the $\beta$ subunit of human TSH and genomic DNA encoding the $\alpha$ subunit of human TSH respectively.

6. A method as claimed in claim 5, wherein the $\beta$ subunit of human thyroid stimulating hormone comprises the sequence set forth in Table 9.

7. A method as claimed in claim 1 wherein the first structural gene comprises at least the first intron (intron I) of genomic TSH $\beta$.

8. A method according to claim 7 wherein the first structural gene does not include the first exon (exon I) of genomic TSH $\beta$.

9. A method for the production of thyroid stimulating hormone comprising the steps of:

a) providing a first vector comprising a promoter, a DNA fragment encoding the $\alpha$ subunit of said thyroid stimulating hormone and comprising at least one intron, and a terminating sequence, and also providing a second vector comprising a promoter and a DNA fragment TSHB 1.2 or TSHB 2.0 encoding the $\beta$ subunit of said thyroid stimulating hormone (as shown in Fig 3);

b) transforming host cells with said vectors; and

c) culturing said transformed cells under conditions whereby said thyroid stimulating hormone is produced.

10. The method of claim 9 wherein said DNA fragment encoding said $\alpha$ subunit further comprises a plurality of introns, 3' and 5' flanking regions, endogenous 5' untranslated sequence and polyadenylation signal.

11. The method of claim 9 or claim 10 wherein said thyroid stimulating hormone is human thyroid stimulating hormone.

12. The method of any of claims 9 to 11 wherein the second vector comprises the DNA fragment THSB 1.2.

**Patentansprüche**

1. Verfahren zur Herstellung eines thyreotropen Hormons (TSH). Es besteht aus folgenden Schritten:

a) Ausstatten eines Vektors mit einem Promotor und einem Strukturgen, wobei zumindest ein Teil der $\beta$-Untereinheit des Hormons kodiert ist. Ebenso ist eine Endsequenz beinhaltet. Der Vektor kann optionsweise auch ein zweites Strukturgen enthalten, wobei zumindest ein Teil der $\alpha$-Untereinheit des TSH kodiert ist und das erste Strukturgen enthält zumindest ein Intron. Das zweite strukturierte Gen (wenn es im Vektor vorhanden ist) enthält mindestens ein Intron.

b) Enthält der Vektor das zweite Strukturgen nicht, wird ein zweiter Vektor, der das zweite Strukturgen enthält, mit mindestens einem Intron ausgestattet.

# EP 0 404 546 B1

c) Transformieren der Wirtszellen mit dem (den) Vektor(en), damit dadurch beständige transfektierte Zellen gebildet werden können.

d) Kultivieren der transformierten Zellen unter solchen Bedingungen, unter welchen sich das thyreotrope Hormon bilden kann.

2. Verfahren wie in Antrag 1, wobei das zweite strukturelle Gen eine cDNA-Sequenz enthält, die die $\alpha$-Untereinheit des TSH kodiert und ein 3'- und ein 5'-Ende hat; das besagte zweite strukturelle Gen enthält ein Intron, welches sich näher dem 5'-Ende als dem 3'-Ende der cDNA-Sequenz befindet.

3. Verfahren nach Antrag 2, wobei die $\alpha$-Untereinheit die Sequenz enthält, die in Tabelle 7 dargestellt ist.

4. Verfahren wie in Antrag 2 oder 3, wobei die cDNA die $\alpha$-Untereinheit kodiert und eine Vielzahl an Introns, 3'-und 5'-Flanken, endogene 5' nicht translatierte Sequenzen und ein Polyadenylierungssignal enthält.

5. Verfahren nach Antrag 1, wobei das thyreotrope Hormon vom Menschen stammt und das erste und zweite Strukturgen eine genomische DNA enthalten, die die $\beta$-Untereinheit des menschlichen TSH kodiert und ebenso eine genomische DNA, die die $\alpha$-Untereinheit des menschlichen Hormons kodiert.

6. Verfahren nach Antrag 5, wobei die $\beta$-Untereinheit des menschlichen thyreotropen Hormons eine Sequenz wie in Tabelle 9 dargestellt enthält.

7. Verfahren nach Antrag 1, wobei das erste strukturelle Gen zumindest das erste Intron (Intron I) des genomischen $\beta$-TSH enthält.

8. Verfahren, nach Antrag 7, wobei das erste Strukturgen das erste Exon (Exon I) des genomischen $\beta$-TSH nicht enthält.

9. Verfahren für die Produktion eines thyreotropen Hormons, welches aus folgenden Produktionsschritten besteht:

a) Ausstatten eines Vektors mit einem Promotor, einem DNA-Fragment, welches die $\alpha$-Untereinheit des Thyreotropins kodiert und zumindest ein Intron und eine Terminationssequenz enthält. Es wird auch ein zweiter Vektor mit einem Promotor und einem A-DNA-Fragment des TSHB 1.2 oder des TSHB 2.0 versehen, welcher die $\beta$-Untereinheit des thyreotropen Hormons kodiert. (siehe Abb. 3).

b) Transformieren der Wirtszellen mit den oben beschriebenen Vektoren und

c) Kultivieren der umgewandelten Zellen unter Bedingungen, die die Produktion des thyreotropen Hormons ermöglichen.

10. Das nach Antrag 9 beschriebene Verfahren, wobei das DNA-Fragment die $\alpha$-Untereinheit kodiert und eine Vielzahl an Introns, 3'-und 5'-Flanken, ebenso wie eine nicht translatierte Sequenz und ein Polyadenylierungssignal besitzt.

11. Verfahren, nach Antrag 9 oder 10, wobei das thyreotrope Hormon vom Menschen stammt.

12. Verfahren, ähnlich den Anträgen 9-11, wobei der zweite Vektor das DNA-Fragment THSB1.2 enthält.

**Revendications**

1. Méthode de production de la Thyréostimuline (TSH), la méthode comprenant les étapes de :

a) fournir un vecteur comprenant un promoteur et un gène de structure codant pour au moins une portion de la sous-unité $\beta$ de la TSH et comprenant une séquence de terminaison, ledit vecteur comprenant aussi éventuellement un deuxième gène de structure codant pour au moins une partie de la sous-unité $\alpha$ de la TSH; pour lequel le premier gène de structure comprend au moins un intron et le deuxième gène de structure (s'il est présent dans ledit vecteur) comprend au moins un intron.

b) si le vecteur ne comprend pas ledit deuxième gène de structure, fournir un deuxième vecteur comprenant ledit deuxième gène de structure avec au moins un intron.

c) transformer des cellules hôtes avec le(s) vecteur(s) pour obtenir ainsi des cellules transfectées de façon stable; et

d) cultiver les cellules transformées dans les conditions adéquates à la production de TSH.

2. Méthode selon la revendication 1, dans laquelle le deuxième gène de structure comprend une séquence d'ADNc codant la sous-unité α de la TSH et possédant une extrémité 3' et une extrémité 5', ledit gène de structure comprenant un intron qui est placé plus près de l'extrémité 5' que de l'extrémité 3' de la séquence d'ADNc.

3. Méthode selon la revendication 2, dans laquelle la sous-unité α comprend la séquence présentée dans le tableau 7.

4. Méthode selon les revendications 2 ou 3, dans laquelle l'ADNc codant pour ladite sous-unité a comprend plusieurs introns, des régions 3' et 5' périphériques, ainsi qu'une séquence endogène 5' non traduite et le signal de polyadénylation.

5. Méthode selon la revendication 1, dans laquelle la thyréostimuline est la thyréostimuline humaine et où le premier et le deuxième gène de structure contiennent respectivement de l'ADN génomique codant pour la sous unité β de la TSH humaine et de l'ADN génomique codant pour la sous-unité a de la TSH humaine.

6. Méthode selon la revendication 5, dans laquelle la sous unité β de la thyréostimuline humaine comprend la séquence présentée dans le tableau 9.

7. Méthode selon la revendication 1, dans laquelle le premier gène de structure comprend au moins le premier intron (intron I) de la TSH-β génomique.

8. Méthode selon la revendication 7, dans laquelle le premier gène de structure n'inclut pas le premier exon (exon I) de la TSH-β génomique.

9. Méthode de production de la thyréostimuline comprenant les étapes de :

a) fournir un premier vecteur comprenant un promoteur, un fragment d'ADN codant pour la sous-unité α de ladite thyréostimuline et comprenant au moins un intron, et une séquence de terminaison, et fournir aussi un deuxième vecteur contenant un promoteur et un fragment d'ADN TSHβ 1.2 ou TSHβ 2.0 codant pour la sous-unité β de ladite thyréostimuline (selon la Fig. 3);

b) transformer des cellules hôtes avec lesdits vecteurs; et

c) cultiver les cellules transformées dans les conditions adéquates à la production de ladite thyréostimuline.

10. Méthode selon la revendication 9, dans laquelle ledit fragment d'ADN codant pour ladite sous-unité α comprend de plus plusieurs introns, des régions 3' et 5' périphériques, une séquence endogène 5' non traduite et le signal de polyadénylation.

11. Méthode selon les revendications 9 ou 10, dans laquelle la thyréostimuline est la thyréostimuline humaine.

12. Méthode selon l'une quelconque des revendications 9 à 11 dans laquelle le deuxième vecteur comprend le fragment d'ADN THBβ 1.2.

# FIGURE 1A

# FIGURE 1B

# FIGURE 2

* - DHFR, ODC or tPA

# FIGURE 3

# FIGURE 4